# EUROPEAN PATENT APPLICATION

(11) **EP 3 597 110 A1**
(43) Date of publication of application: **22.01.2020**
(21) Application number: 18184433.3
(22) Date of filing: 19.07.2018
(51) Int. Cl.: A61B 6/04, A61B 5/055

(54) **PATIENT SUPPORT COUCH ASSEMBLY AND DIAGNOSTIC IMAGING SYSTEM**

(71) Applicant: Koninklijke Philips N.V., 5656 AE Eindhoven (NL)
(72) Inventor: GUO, Charlie Zg, 5656 AE Eindhoven (NL)
(74) Representative: de Haan, Poul Erik

(57) **Abstract**

The present invention provides a patient support couch assembly comprises a couch panel for supporting a patient, a horizontal frame for supporting the couch panel, a first sliding assembly, a lifting frame for supporting the horizontal frame to move up and down, a second sliding assembly, and a third sliding assembly. Both the first sliding assembly and the second sliding assembly for enabling the horizontal movement of the patient lying on the couch panel are implemented on horizontal faces of the couch panel, the horizontal frame and the top part of the lifting frame separately. The third sliding assembly for enabling the vertical movement of the patient lying on the couch panel are implemented on the vertical faces of the top part and scissor structure support. According to the present invention, the patient support couch assembly is designed in a compact structure to well support the double-layer structure and the sliding performance of the patient couch assembly can still be well supported.

## Description

### FIELD OF THE INVENTION

The invention relates to patient support structure. More particularly, the present invention relates to a patient support couch assembly adapted to be used with a diagnostic imaging apparatus such as a computerized tomography (CT) apparatus, a magnetic resonance imaging apparatus or the like. The present invention also relates to a diagnostic imaging system comprising the patient support couch assembly.

### BACKGROUND OF THE INVENTION

In order to obtain diagnostic images of a patient with a diagnostic imaging apparatus, it is necessary that the patient is located exactly at a predetermined position inside an opening of a scan gantry of the diagnostic imaging apparatus. For this reason, the diagnostic imaging apparatus has been provided with a patient support couch assembly which is movable vertically to be in line with the scan gantry, and horizontally in and out of the scan gantry.

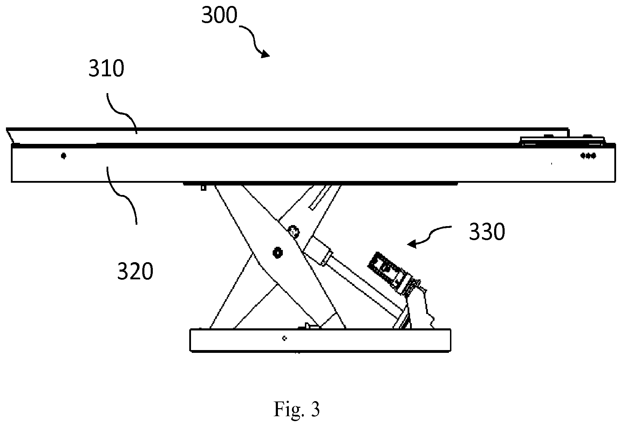

A single-layer patient support couch assembly with a sliding couch panel can enable the patient to be moved horizontally in and out of the scan gantry. In order to increase the horizontal movement range of the patient, a double-layer patient support couch assembly 300 as shown in Figure 3 has been developed with a couch panel layer 310 and a horizontal frame layer 320. The couch panel layer 310 can move horizontally relative to the horizontal frame layer 320 and the horizontal frame layer 320 can move horizontally relative to the couch support 330. The double-layer structure increases the horizontal movement range of the patient lying on the couch.

However, compared with the single-layer patient support couch assembly, an additional movable layer of the double-layer patient support couch assembly may increase the size of the patient support couch assembly and it may be difficult to increase the size of the elevators or vehicles for carrying the patent support couch assembly. Thus, there is a need to provide a double-layer patent support couch assembly which does not occupy much more space than the single-layer patient support couch assembly.

### SUMMARY OF THE INVENTION

It would be advantageous to achieve a patent support couch assembly having a double-layer structure with good horizontal movement performance. It would also be desirable to have the size of the patent support couch assembly as small as possible.

To better address one or more of the above concerns, according to a first aspect of the present invention, it is to provide a patient support couch assembly comprising:
a couch panel for supporting a patient;
a horizontal frame for supporting the couch panel;
a first sliding assembly implemented between a first lower face of the couch panel and a first upper face of the horizontal frame for enabling the couch panel to slide horizontally relative to the horizontal frame;
a lifting frame for supporting the horizontal frame to move up and down, the lifting frame comprising a top part and a scissor structure support;
a second sliding assembly implemented between a first horizontal face of the horizontal frame and a second horizontal face of the top part for enabling the horizontal frame to slide horizontally relative to the top part;
a third sliding assembly implemented between a first vertical face of the top part and a second vertical face of a first upper end of the scissor structure support for enabling the first upper end of the scissor structure support to slide horizontally relative to the top part.

Both the first sliding assembly and the second sliding assembly for enabling the horizontal movement of the patient lying on the couch panel are implemented on horizontal faces of the couch panel, the horizontal frame and the top part of the lifting frame separately. The third sliding assembly for enabling the vertical movement of the patient lying on the couch panel is implemented on the vertical faces of the top part and scissor structure support. The required extra spaces for installing the sliding assemblies are well arranged to obtain a good balance in the vertical direction and horizontal direction without increasing the height and width of the patient support couch assembly too much. If the border parts of the couch panel have already been folded upwards for the single-layer patient couch assembly, there will be no need to require extra space for installing the first sliding assembly and the second sliding assembly. Only the third sliding assembly increases the width of the patient support couch, so there will still be enough space to enable a wide enough lifting frame to steadily support the patient even if the size of the patient support couch assembly is limited by the space of the hospital elevator or vehicles for carrying the patient couch assembly. As a result, the patient support couch assembly is designed in a compact structure to well support the double-layer structure and the sliding performance of the patient couch assembly can still be well supported.

According to a second aspect of the present invention, it is to provide a diagnostic imaging system comprising a diagnostic imaging apparatus and the patient support couch assembly above-mentioned.

According to an embodiment of the present invention, the first horizontal face of the horizontal frame is a second lower face of the horizontal frame, the second lower face of the horizontal frame is opposite to the first upper face of the horizontal frame, the second horizontal face of the top part is a second upper face of the top part. That is to say, the first sliding assembly and the second sliding assembly are installed separately on two sides of a horizontal part of the horizontal frame. In this way, the horizontal frame can have only one horizontal part, i.e., the longitudinal section of the horizontal frame can be in an open shape such as an L shape or a T shape, etc. Compared with other kinds of horizontal frame longitudinal section shape, such as U shape, the open shape will be helpful for manufacturing second sliding assembly with high precision, especially when the second sliding assembly comprises a linear guide track implemented on the horizontal frame.

These and other objects, features and characteristics of the present invention, will become more apparent upon consideration of the following description and the appended claims with reference to the accompanying drawings, all of which form a part of this specification, wherein like reference numerals designate corresponding parts in the various figures. It is to be expressly understood, however, that the drawings are for the purpose of illustration and description only and are not intended as a definition of the limits of the invention.

### BRIEF DESCRIPTION OF THE DRAWINGS

Fig.1a is a perspective view showing a patient support couch assembly according to an embodiment of the present invention.
Fig.1b is a perspective view of a part of the patient support couch assembly shown in FIG. 1a with a portion of the patient support couch assembly being cut off to show clearly the structure of the patient support couch assembly.
Fig.1c is a perspective view showing the horizontal frame of the patient support couch assembly shown in Fig. 1b.
Fig.1d is a perspective view showing the lifting frame of patient support couch assembly shown in Fig. 1a.
Fig.1e is a perspective view showing the top part of the lifting frame shown in Fig.1d.
Fig. 1f is a perspective view showing a side view of the patient support couch assembly shown in Fig.1b.
Fig.2a is a perspective view showing a part of a patient support couch assembly according to another embodiment of the present invention with a portion of the patient support couch assembly being cut off to show clearly the structure of the patient support couch assembly.
Fig.2b is a perspective view showing a side view of the patient support couch assembly shown in FIG.2a.
Fig.3 is a perspective view showing a double layer patient support couch assembly.

The same reference numerals are used to denote similar parts throughout the figures.

### DETAILED DESCRIPTION OF THE EXEMPLARY EMBODIMENTS

Detailed description of the present invention is given below in connection with the accompanying drawings.

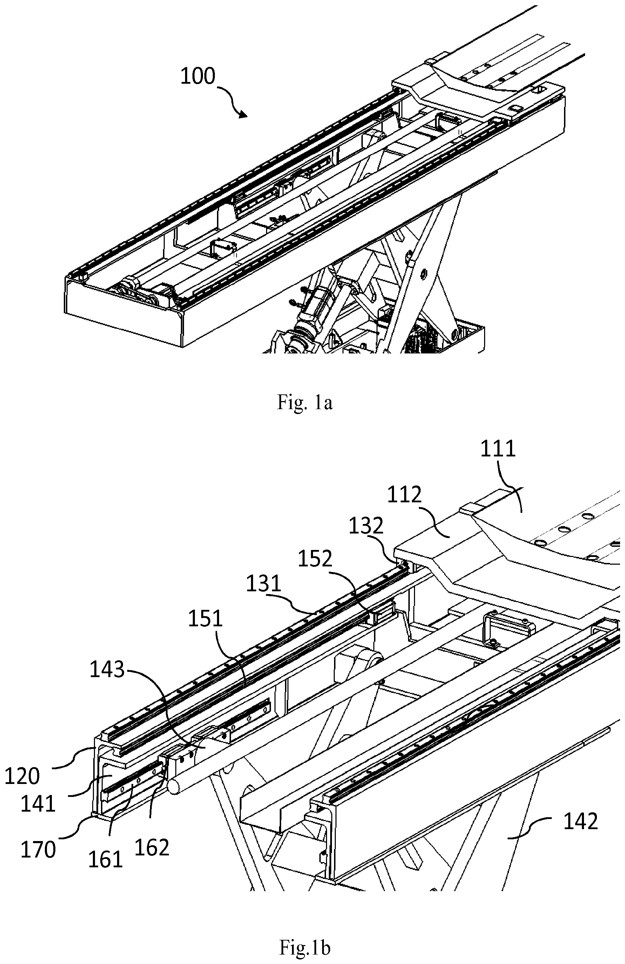

Fig.1a is a perspective view showing a patient support couch assembly 100 according to an embodiment of the present invention. Fig.1b is a perspective view of a part of the patient support couch assembly 100 shown in FIG.1a with a portion of the patient support couch assembly 100 being cut off to show clearly the structure of the patient support couch assembly 100.

As shown in Fig.1a and Fig.1b, the patient support couch assembly 100 comprises a couch panel 111, 112 for supporting a patient, a horizontal frame 120 for supporting the couch panel 111, 112, a first sliding assembly 131, 132, a lifting frame for supporting the horizontal frame 120 to move up and down, a second sliding assembly 151, 152, and a third sliding assembly 161, 162.

During the examination, a patient will lie on the couch panel 111, 112 and the couch panel 111, 112 can be implemented in many ways. In an embodiment as shown in Fig.1a and Fig.1b, the couch panel 111, 112 comprises a carbon top 111 for supporting the patient and a carbon base 112 supported by the horizontal frame 120. The carbon top 111 is fixed on the top of the carbon base 112. In another embodiment (not shown), the couch panel 111, 112 can be a single board for supporting the patient and the single board is supported by the horizontal frame 120. In a further embodiment (not shown), the couch panel 111, 112 may have more than two layers to enable a more comfortable experience for the patient to lie on the couch panel 111, 112.

The horizontal frame 120 is implemented under the couch panel 111, 112 to support the couch panel 111, 112, and the couch panel 111, 112 can slide horizontally relative to the horizontal frame 120 via the first sliding assembly 131, 132 implemented between the couch panel 111, 112 and the horizontal frame 120.

The first sliding assembly 131, 132 is implemented between a first lower face of the couch panel 111, 112 and a first upper face of the horizontal frame 120 for enabling the couch panel 111, 112 to slide horizontally relative to the horizontal frame 120. The first lower face is the lower face of a part of the couch panel 111, 112 and the first upper face is the upper face of a part of the horizontal frame 120. The first lower face of the couch panel 111, 112 is opposite to the first upper face of the horizontal frame 120.

The couch panel 111, 112 or a component (such as said carbon base 112) of the couch panel 111 can be in different kinds of shape, such as a flat shape or a multi-zigzag shape, etc. As shown in Fig. 1a and Fig.1b, the carbon base 112 of the couch panel 111, 112 is in a multi-zigzag shape with five parts. The first lower face is the lower face of a border part of the carbon base 112.

The first sliding assembly 131, 132 can be implemented in many ways. In an embodiment as shown in Fig.1b, the first sliding assembly 131, 132 comprises a first linear guide rail 131 implemented on the first upper face of the horizontal frame 120 and a first sliding block 132 implemented on the first lower face of the couch panel 111. The first sliding block 132 is fastened to the first linear guide rail 131 and is able to slide along the first linear guide rail 131. In another embodiment (not shown), the first sliding assembly comprise a sliding block implemented on the first upper face of the horizontal frame and a linear guide rail implemented on the first lower face of the couch panel.

The linear guide rail 131 can be in many kinds of shapes, such as a T shape, an L shape guide rail, a U shape guide rail and an O shape guide rail, etc. In an embodiment as shown in Fig.1b, the linear guide rail 131 is in a T shape. For linear guide rails in different kinds of shapes, the sliding blocks may be different. It is known to the people skilled in the art how to implement different types of linear guide rails and sliding blocks. Therefore, this will not be further elaborated here. Furthermore, it is known to the people skilled in the art how to drive the movement of the first sliding assembly, such as by a motor, and it will not be elaborated here.

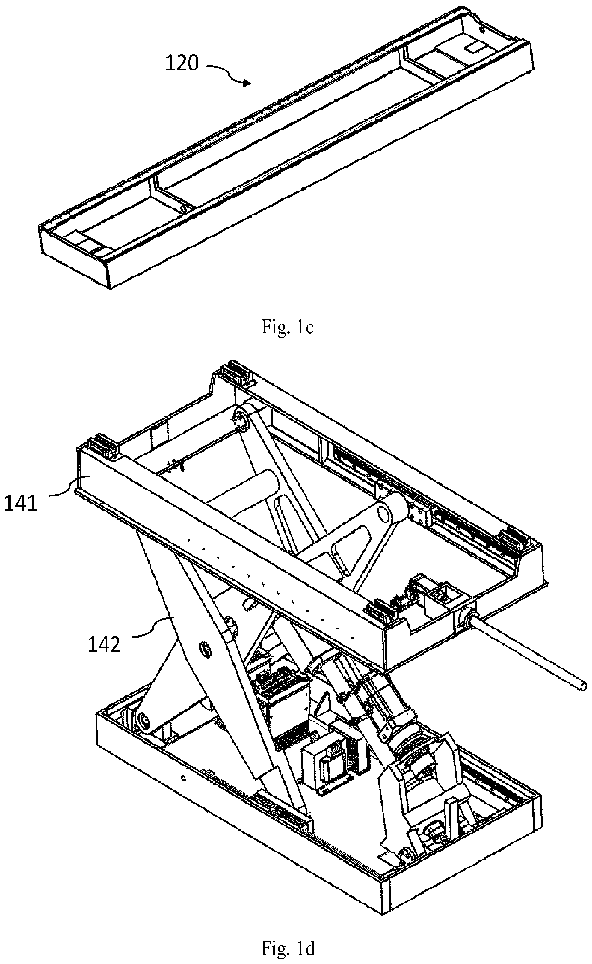

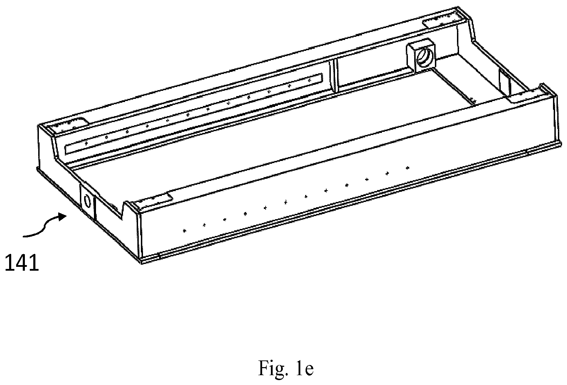

Fig.1d is a perspective view showing the lifting frame of patient support couch assembly 100 shown in Fig. 1a. Fig.1e is a perspective view showing the top part 141 of the lifting frame shown in Fig.1d. As shown in Fig.1b, Fig.1e and Fig.1d, the lifting frame comprises a top part 141 and a scissor structure support 142. The top part 141 can support the horizontal frame 120. The scissor structure support 142 includes two legs and the two legs are secured together at an intermediate portion of each leg. The relative movement between the two legs of the scissor structure support 142 will change the height of the top part 141, i.e. the height of the horizontal frame 120 and the height of the couch panel 111, 112.

The second sliding assembly 151, 152 is implemented between a first horizontal face of the horizontal frame 120 and a second horizontal face of the top part 141 for enabling the horizontal frame 120 to slide horizontally relative to the top part 141. The first horizontal face is a horizontal face of a part of the horizontal frame 120 and the second horizontal face is a horizontal face of a part of the top part. The first horizontal face of the horizontal frame is opposite to the second horizontal face of the top part.

Fig.1c is a perspective view showing the horizontal frame 120 of the patient support couch assembly 100 shown in Fig. 1b. In an embodiment of the horizontal frame 120 as shown in Fig.1b and Fig.1c, the first horizontal face of the horizontal frame 120 is a second lower face of the horizontal frame 120, the second lower face of the horizontal frame 120 is opposite to the first upper face of the horizontal frame 120, the second horizontal face of the top part 141 is a second upper face of the top part 141. In this way, the first horizontal face (i.e. the second lower face) of the horizontal frame 120 and the first upper face of the horizontal frame are two sides of the same part of the horizontal frame, and then the longitudinal section of the horizontal frame can be in an open shape, such as an L shape or a T shape, etc. With the open shape of the horizontal frame longitudinal section, the second sliding assembly 151, 152 can be implemented on the horizontal frame 120 with high precision which is important to the slide movement of the patient support couch assembly 100. As shown in Fig.1b, the longitudinal section of the horizontal frame 120 is in an L shape. The longitudinal section of the top part 141 is also in an L shape. A stiffener board 170 is welded to the top part 141 for reinforcement.

The second sliding assembly 151, 152 can be implemented in many ways. In an embodiment as shown in Fig.1b, the second sliding assembly 151, 152 comprises a second linear guide rail 151 implemented on the first horizontal face (i.e. the second lower face) of the horizontal frame 120 and a second sliding block 152 implemented on the second horizontal face of the top part 141. The second sliding block 152 is fastened to the second linear guide rail 151 and is able to slide along the second linear guide rail 151. In another embodiment (not shown), the second sliding assembly comprise a sliding block implemented on the first horizontal face (i.e. the second lower face) of the horizontal frame and a linear guide rail implemented on the second horizontal face of the top part.

Similar to the first sliding assembly, it is known to the people skilled in the art how to implement different types of linear guide rails and sliding blocks, and it will not be elaborated here. Furthermore, it is known to the people skilled in the art how to drive the movement of the second sliding assembly, such as by a motor, and it will not be elaborated here.

The third sliding assembly 161, 162 implemented between a first vertical face of the top part 141 and a second vertical face of a first upper end 143 of the scissor structure support 142 for enabling the first upper end 143 of the scissor structure support 142 to slide horizontally relative to the top part 141. The first vertical face of is a vertical face of a part of the top part 141 and the second vertical face is a vertical face of a part of the first upper end of the scissor structure support 142. The first vertical face of the top part 141 is opposite to the second vertical face of a first upper end 143 of the scissor structure support 142. The scissor structure support 142 has two legs, and the first upper end 143 is the upper/top end of one leg of the scissor structure support 142.

The third sliding assembly 161, 162 can be implemented in many ways. In an embodiment as shown in Fig.1b, the third sliding assembly 161, 162 comprises the third linear guide rail 161 implemented on the first vertical face of the top part 141 and a third sliding block 162 implemented on the second vertical face of the first upper end 143 of the scissor structure support 142. The third sliding block 162 is fastened to the third linear guide rail 161 and is able to slide along the third linear guide rail 161. In another embodiment (not shown), the third sliding assembly comprise a sliding block implemented on the first vertical face of the top part and a linear guide rail implemented on the second vertical face of the first upper end of the scissor structure support. When the linear guide rail is implemented on the second vertical face of the first upper end of the scissor structure support, the length of the guide rail may be limited by the area of the first upper end of the scissor structure support and thus the movement range of the scissor support structure may be limited.

Similar to the first sliding assembly 131, 132, it is known to the people skilled in the art how to implement different types of linear guide rails and sliding blocks, and it will not be elaborated here. Furthermore, it is known to the people skilled in the art how to drive the movement of the third sliding assembly, such as by a motor, and it will not be elaborated here.

The longitudinal section the horizontal frame 120 can be in different kinds of shape, such as an L shape, a U shape or a T shape, etc. Fig. 1c is a perspective view showing the horizontal frame of the patient support couch assembly shown in Fig. 1a. According to an embodiment of the horizontal frame 120 as shown in Fig.1a, Fig.1b and Fig.1c, the longitudinal section the horizontal frame 120 is in an L shape.

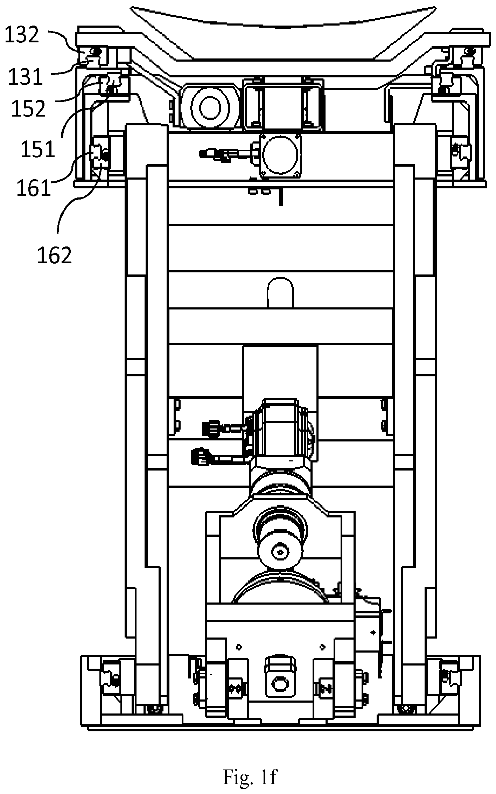

Fig. 1f is a perspective view showing a side view of the patient support couch assembly 100 shown in Fig.1b. As shown in Fig.1f, the first sliding assembly 131, 132, the second sliding assembly 151, 152 and the third sliding assembly 161, 162 are implemented in a compact structure. It is usually folded upwards for the border parts of the couch panel 111, 112 of the single layer patient couch assembly. The first sliding assembly 131, 32 and the second sliding assembly 151, 152 implemented to support a double layer patient couch assembly may use the existing space under the upwards folded border parts of the couch panel, and only the third sliding assembly 161, 162 uses extra space compared with the single layer patient support couch assembly. Therefore, the width of the lifting frame will not be reduced too much if the double layer patient couch patient assembly should keep the same width as that of the single layer patient couch assembly, and thus the stability of the double layer patient couch assembly can be maintained.

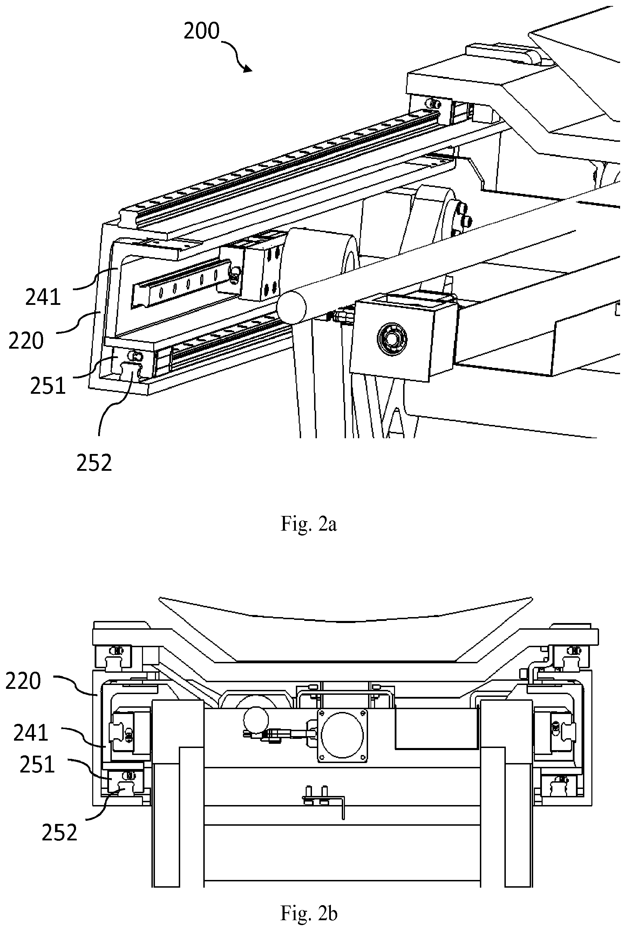

Fig.2a is a perspective view showing a part of a patient support couch assembly 200 according to another embodiment of the present invention with a portion of the patient support couch assembly 200 being cut off to show clearly the structure of the patient support couch assembly 200. Fig.2b is a perspective view showing a side view of the patient support couch assembly 200 shown in FIG.2a.

The first horizontal face of the horizontal frame 220 is a third upper face of the horizontal frame 220, the second horizontal face of the top part 241 is a third lower face of the top part 241.

As shown in Fig.2a, it is similar to the embodiment shown in Fig.1b for the implementation of the first sliding means for enabling the relative sliding movement between the couch panel and the horizontal frame and the third sliding means for enabling the relative sliding movement between the horizontal frame and the top part of the lifting frame.

Different from the embodiment shown in Fig. 1b, both the longitudinal section of the horizontal frame 220 and the longitudinal section of the top part 241 are in a U shape in the embodiment shown in Fig.2a. Because the U shape is not an open shape, it will not be as easy as the open shape for the manufacture of the second sliding assembly on the horizontal frame and the manufacture of the third sliding assembly on the top part.

The second sliding assembly comprised a fourth linear guide rail 251 implemented on the first horizontal face (i.e. the third upper face) of the horizontal frame 220 and a fourth sliding block 252 implemented on the second horizontal face (i.e. the third lower face) of the top part 241. The fourth sliding block 252 is fastened to the fourth linear guide rail 251 and is able to slide along the fourth linear guide rail 251.

The above mentioned different embodiments of the patient couch assembly can be comprised in a diagnostic imaging system. Preferably, such a diagnostic imaging system comprises a diagnostic imaging apparatus in which any embodiment of the patient couch assembly may be comprised.

Although the invention has been described in detail for the purpose of illustration based on what is currently considered to be the most practical and preferred embodiments, it is to be understood that such detail is solely for that purpose and that the invention is not limited to the disclosed embodiments, but on the contrary, is intended to cover modifications and equivalent arrangements that are within the spirit and scope of the appended claims.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measured cannot be used to advantage. Any reference signs in the claims should not be construed as limiting the scope.

## Claims

1. A patient support couch assembly (100, 200, 300) comprising:
a couch panel (111, 112) for supporting a patient;
a horizontal frame (120, 220) for supporting the couch panel (111, 112);
a first sliding assembly (131, 132) implemented between a first lower face of the couch panel (111, 112) and a first upper face of the horizontal frame (120) for enabling the couch panel (111, 112) to slide horizontally relative to the horizontal frame (120);
a lifting frame for supporting the horizontal frame (120) to move up and down, the lifting frame comprising a top part (141, 241) and a scissor structure support (142);
a second sliding assembly (151, 152, 251, 252) implemented between a first horizontal face of the horizontal frame (120) and a second horizontal face of the top part (141) for enabling the horizontal frame (120) to slide horizontally relative to the top part (141);
a third sliding assembly (161, 162) implemented between a first vertical face of the top part (141) and a second vertical face of a first upper end (143) of the scissor structure support (142) for enabling the first upper end (143) of the scissor structure support (142) to slide horizontally relative to the top part (141).

2. The patient support couch assembly (100, 200, 300) according to claims 1, wherein the first horizontal face of the horizontal frame (120) is a second lower face of the horizontal frame (120), the second lower face of the horizontal frame (120) is opposite to the first upper face of the horizontal frame (120), the second horizontal face of the top part (141) is a second upper face of the top part (141).

3. The patient support couch assembly (100, 200, 300) according to claims 1, wherein the first horizontal face of the horizontal frame (220) is a third upper face of the horizontal frame (220), the second horizontal face of the top part (241) is a third lower face of the top part (241).

4. The patient support couch assembly (100, 200, 300) according to any one of claims 1-3, wherein the couch panel (111, 112) comprises a carbon top (111) for supporting the patient and a carbon base (112) supported by the horizontal frame (120, 220).

5. The patient support couch assembly (100, 200, 300) according to any one of claims 1 to 4, wherein the first sliding assembly (131, 132) comprised a first linear guide rail (131) implemented on the first upper face of the horizontal frame (120, 220) and a first sliding block (132) implemented on the first lower face of the couch panel (111), the first sliding block (132) is fastened to the first linear guide rail (131) and is able to slide along the first linear guide rail (131).

6. The patient support couch assembly (100, 200, 300) according to claims 1, 2, 4 or 5, wherein the second sliding assembly (151, 152) comprised a second linear guide rail (151) implemented on the first horizontal face of the horizontal frame (120) and a second sliding block (152) implemented on the second horizontal face of the top part (141), the second sliding block (152) is fastened to the second linear guide rail (151) and is able to slide along the second linear guide rail (151).

7. The patient support couch assembly (100, 200, 300) according to claims 1, 3, 4 or 5, wherein the second sliding assembly (251, 252) comprised a fourth linear guide rail (251) implemented on the first horizontal face of the horizontal frame (220) and a fourth sliding block (252) implemented on the second horizontal face of the top part (241), the fourth sliding block (252) is fastened to the fourth linear guide rail (251) and is able to slide along the fourth linear guide rail (251).

8. The patient support couch assembly (100, 200, 300) according to any one of claims 1 to 7, wherein the third sliding assembly (161, 162) comprises a third linear guide rail (161) implemented on the first vertical face of the top part (141, 241) and a third sliding block (162) implemented on the second vertical face of the first upper end (143) of the scissor structure support (142), the third sliding block (162) is fastened to the third linear guide rail (161) and is able to slide along the third linear guide rail (161).

9. The patient support couch assembly (100, 200, 300) according to any one of claims 1 to 8, wherein at least one of the first linear guide rail (131), the second linear guide rail (151), the third linear guide rail (162) and/or the fourth linear guide rail (251) is one or combination of a T shape linear guide rail, an L shape linear guide rail, a U shape linear guide rail and an O shape linear guide rail.

10. A diagnostic imaging system comprising a diagnostic imaging apparatus and a patient support couch assembly (100, 200, 300) according to any one of claims 1- 9.
